(19) Europäisches Patentamt
European Patent Office
Office européen des brevets

(11) Veröffentlichungsnummer: 0 347 789
A2

(12) **EUROPÄISCHE PATENTANMELDUNG**

(21) Anmeldenummer: 89111069.4

(51) Int. Cl.[4]: C07D 327/04 , A01N 43/28

(22) Anmeldetag: 19.06.89

(30) Priorität: 22.06.88 DE 3821553

(43) Veröffentlichungstag der Anmeldung:
27.12.89 Patentblatt 89/52

(84) Benannte Vertragsstaaten:
AT BE CH DE ES FR GB GR IT LI LU NL SE

(71) Anmelder: SCHERING AKTIENGESELLSCHAFT
Berlin und Bergkamen
Müllerstrasse 170/178 Postfach 65 03 11
D-1000 Berlin 65(DE)

(72) Erfinder: Franke, Wilfried, Dr.
Spiessergasse 6b
D-1000 Berlin 27(DE)
Erfinder: Dorfmeister, Gabriele, Dr.
Heiligenseestrasse 70
D-1000 Berlin 27(DE)
Erfinder: Ganzer, Michael, Dr.
Dannenwalder Weg 14
D-1000 Berlin 26(DE)
Erfinder: Johann, Gerhard, Dr.
Hermsdorfer Damm 147
D-1000 Berlin 28(DE)
Erfinder: Rees, Richard, Dr.
Speerweg 8
D-1000 Berlin 28(DE)

(54) **Substituierte N-Phenyl-4-alkyliden-1,3-oxathiolan-2-imine, Verfahren zu ihrer Herstellung und ihre Verwendung als Mittel mit herbizider Wirkung.**

(57) Die Erfindung betrifft neue substituierte N-Phenyl-4-alkyliden-1,3-oxathiolan-2-imine der allgemeinen Formel I

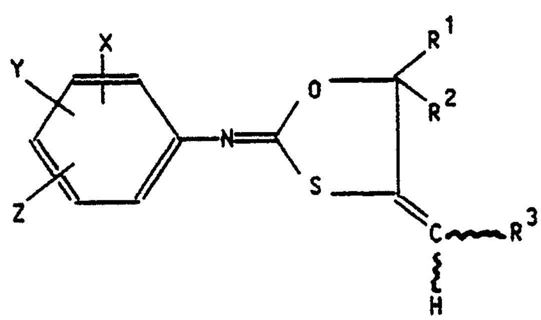

(I) ,

in der X, Y, Z, $R^1$, $R^2$ und $R^3$ die in der Beschreibung genannten Bedeutungen haben, und deren Salze mit anorganischen und organischen Säuren, Verfahren zu ihrer Herstellung und ihre Verwendung als Mittel mit herbizider Wirkung.

EP 0 347 789 A2

# SUBSTITUIERTE N-PHENYL-4-ALKYLIDEN-1,3-OXATHIOLAN-2-IMINE, VERFAHREN ZU IHRER HERSTELLUNG UND IHRE VERWENDUNG ALS MITTEL MIT HERBIZIDER WIRKUNG

Die Erfindung betrifft neue substituierte N-Phenyl-4-alkyliden-1,3-oxathiolan-2-imine, Verfahren zu ihrer Herstellung und ihre Verwendung als Mittel mit herbizider Wirkung.

Es ist bereits bekannt, daß 4-Methylen-1,3-oxathiolane herbizide Eigenschaften besitzen (DE-OS 36 01 048). Häufig ist jedoch die Herbizidwirkung der bekannten Verbindungen nicht ausreichend, oder es treten Selektivitätsprobleme in wichtigen landwirtschaftlichen Kulturen auf.

Aufgabe der vorliegenden Erfindung ist daher die Bereitstellung von neuen Verbindungen, die diese Nachteile nicht aufweisen und in ihren biologischen Eigenschaften den bisher bekannten Verbindungen überlegen sind.

Es wurde gefunden, daß substituierte N-Phenyl-4-alkyliden-1,3-oxathiolan-2-imine der allgemeinen Formel I

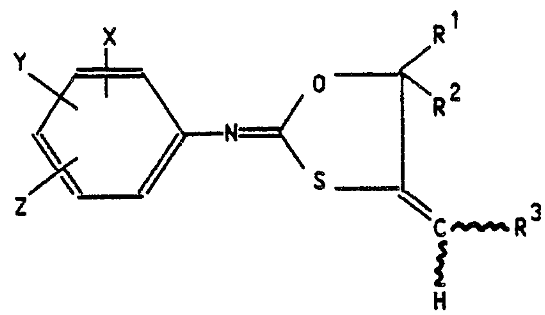

(I) ,

in der

X ein Halogenatom oder einen Halogen-$C_1$-$C_4$-alkylrest,

Y ein Wasserstoffatom, einen $C_1$-$C_4$-Alkylrest, einen Halogen-$C_1$-$C_4$-alkylrest, einen $C_1$-$C_4$-Alkoxyrest, einen Halogen-$C_1$-$C_4$-alkoxyrest, ein Halogenatom oder eine Nitrogruppe,

Z ein Wasserstoffatom, einen $C_1$-$C_4$-Alkylrest, einen Halogen-$C_1$-$C_4$-alkylrest, einen $C_1$-$C_4$-Alkoxyrest, einen Halogen-$C_1$-$C_4$-alkoxyrest, ein Halogenatom oder eine Nitrogruppe,

$R^1$ ein Wasserstoffatom, einen $C_1$-$C_4$-Alkylrest, einen Halogen-$C_1$-$C_4$-alkylrest oder einen Phenylrest,

$R^2$ ein Wasserstoffatom, einen $C_1$-$C_4$-Alkylrest, einen Halogen-$C_1$-$C_4$-alkylrest oder einen Phenylrest oder

$R^1$ und $R^2$ gemeinsam mit dem benachbarten Kohlenstoffatom einen carbocyclischen $C_3$-$C_6$-Ring bilden und

$R^3$ ein Wasserstoffatom, einen $C_1$-$C_4$-Alkylrest, einen Halogen-$C_1$-$C_4$-alkylrest oder einen Phenylrest

bedeuten, und deren Salze mit anorganischen und organischen Säuren eine interessante herbizide Wirkung zeigen.

Die Verbindungen der allgemeinen Formel I können gegebenenfalls in verschiedenen enatiomeren, diastereomeren oder geometrischen Formen anfallen und sind ebenfalls Gegenstand dieser Erfindung.

Die Bezeichnung "Halogen" umfaßt Fluor, Chlor, Brom und Jod.

Die Bezeichnung "Halogenalkyl" besagt, daß ein oder mehrere Wasserstoffatome des Alkylrestes durch Halogen ersetzt sind.

Die erfindungsgemäßen Verbindungen der allgemeinen Formel I lassen sich herstellen, indem man Verbindungen der allgemeinen Formel II

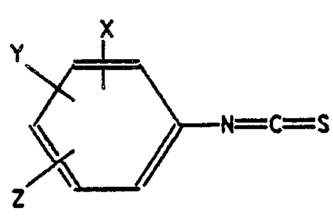

(II) ,

in der X, Y und Z die unter der allgemeinen Formel I genannten Bedeutungen haben, mit Verbindungen der allgemeinen Formel III

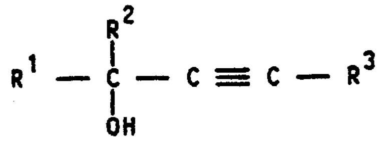 (III) ,

in der $R^1$, $R^2$ und $R^3$ die unter der allgemeinen Formel I genannten Bedeutungen haben, unter Verwendung eines basischen Katalysators umsetzt.

Die genannten Ausgangsmaterialien sind bekannt oder lassen sich analog zu bekannten Verfahren herstellen.

Das Verfahren wird zweckmäßigerweise so durchgeführt, daß man die Ausgangsverbindungen der allgemeinen Formeln II und III in einem geeigneten Lösungsmittel unter Zusatz katalytischer bis äquimolarer Mengen einer anorganischen Base über längere Zeit, wie zum Beispiel 0,5 bis 15 Stunden, bei einer Temperatur von 20°C bis zur Siedetemperatur des jeweiligen Lösungsmittels, gewöhnlich aber bei Raumtemperatur umsetzt.

Als basische Katalysatoren können zum Beispiel Alkali- oder Erdalkalihydroxide, Alkoholate und Alkalihydride eingesetzt werden. Beispielhaft seien Natrium- und Kaliumhydroxid, Natriummethanolat und Natriumhydrid genannt.

Als Lösungsmittel kommen Kohlenwasserstoffe, wie zum Beispiel Toluol, chlorierte Kohlenwasserstoffe, wie zum Beispiel Methylenchlorid oder Chloroform, Ether, wie zum Beispiel Diethylether oder Tetrahydrofuran, Alkohole, wie zum Beispiel Methanol oder Ethanol, Ketone, wie zum Beispiel Aceton oder Butanon, Amide, wie zum Beispiel Dimethylformamid, oder auch Sulfoxide, wie zum Beispiel Dimethylsulfoxid, in Frage.

Die Säureadditionssalze lassen sich herstellen, indem man eine Verbindung der allgemeinen Formel I mit mindestens einem Äquivalent einer anorganischen oder organischen Säure gegebenenfalls in einem der oben angegebenen Lösungsmittel umsetzt. Als Säuren seien zum Beispiel genannt Chlorwasserstoffsäure, Bromwasserstoffsäure, Salpetersäure, Schwefelsäure, Essigsäure oder Methansulfonsäure.

Die Aufarbeitung der erfindungsgemäßen Verbindungen erfolgt in der üblichen Art und Weise. Eine Aufreinigung oder gegebenenfalls auch Trennung anfallender isomerer Verbindungen erfolgt durch Kristallisation oder Säulenchromatographie.

Die erfindungsgemäßen Verbindungen stellen in der Regel farblose bis gelb gefärbte kristalline oder zähflüssige Substanzen dar, die zum Teil gut löslich in halogenierten Kohlenwasserstoffen, wie zum Beispiel Methylenchlorid oder Chloroform, Sulfoxiden, wie zum Beispiel Dimethylsulfoxid, Estern, wie zum Beispiel Essigester, Ethern, wie zum Beispiel Diethylether oder Tetrahydrofuran, oder Alkoholen, wie zum Beispiel Methanol oder Ethanol, sind.

Die erfindungsgemäßen Wirkstoffe zeigen eine gute herbizide Wirkung bei breitblättrigen Unkräutern und Gräsern. Ein selektiver Einsatz der erfindungsgemäßen Wirkstoffe ist in verschiedenen Kulturen möglich, zum Beispiel in Raps, Rüben, Sojabohnen, Baumwolle, Reis, Gerste, Weizen und anderen Getreidearten. Dabei sind einzelne Wirkstoffe als Selektivherbizide in Rüben, Baumwolle, Soja und Getreide besonders geeignet. Ebenso können die Verbindungen zur Unkrautbekämpfung in Dauerkulturen, zum Beispiel Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, und zur selektiven Unkrautbekämpfung in einjährigen Kulturen eingesetzt werden. Die erfindungsgemäßen Wirkstoffe können auch als Defoliants, als Desiccants und als Krautabtötungsmittel verwendet werden.

Die erfindungsgemäßen Wirkstoffe können zum Beispiel bei den folgenden Pflanzengattungen verwendet werden:

Dikotyle Unkräuter der Gattungen Sinapis, Lepidium, Galium, Stellaria, Matricaria, Anthemis, Galinsoga, Chenopodium, Brassica, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Lamium, Veronica, Abutilon, Datura, Viola, Galeopsis, Papaver, Centaurea und Chrysanthemum.

Monokotyle Unkräuter der Gattungen Avena, Alopecurus, Echinochloa, Setaria, Panicum, Digitaria, Poa, Eleusine, Brachiaria, Lolium, Bromus, Cyperus, Agropyron, Sagittaria, Monocharia, Fimbristylis, Eleocharis, Ischaemum und Apera.

Die Aufwandmengen schwanken je nach Anwendungsart im Vor- und Nachauflauf in den Grenzen zwischen 0,05 und 5 kg/ha.

Die erfindungsgemäßen Verbindungen können entweder allein, in Mischung miteinander oder mit anderen Wirkstoffen angewendet werden. Gegebenenfalls können andere Pflanzenschutz- oder Schädlingsbekämpfungsmittel je nach dem gewünschten Zweck zugesetzt werden. Sofern eine Verbreiterung des

Wirkungsspektrums beabsichtigt ist, können auch andere Herbizide zugesetzt werden. Beispielsweise eignen sich als herbizid wirksame Mischungspartner diejeni gen Wirkstoffe, die in Weed Abstracts, Vol. 36, No. 12, 1987, unter dem Titel "List of common names and abbreviations employed for currently used herbicides and plant growth regulators in Weed Abstracts" aufgeführt sind.

Eine Förderung der Wirkintensität und der Wirkungsgeschwindigkeit kann zum Beispiel durch wirkungssteigernde Zusätze, wie organische Lösungsmittel, Netzmittel und Öle, erzielt werden. Solche Zusätze lassen daher gegebenenfalls eine Verringerung der Wirkstoffdosierung zu.

Zweckmäßig werden die gekennzeichneten Wirkstoffe oder deren Mischungen in Form von Zubereitungen wie Pulvern, Streumitteln, Granulaten, Lösungen, Emulsionen oder Suspensionen, unter Zusatz von flüssigen und/oder festen Trägerstoffen beziehungsweise Verdünnungsmitteln und gegebenenfalls Haft-, Netz-, Emulgier- und/oder Dispergierhilfsmitteln angewandt.

Geeignete flüssige Trägerstoffe sind zum Beispiel aliphatische und aromatische Kohlenwasserstoffe wie Benzol, Toluol, Xylol, Cyclohexanon, Isophoron, Dimethylsulfoxid, Dimethylformamid, weiterhin Mineralölfraktionen und Pflanzenöle.

Als feste Trägerstoffe eignen sich Mineralien, zum Beispiel Bentonit, Silicagel, Talkum, Kaolin, Attapulgit, Kalkstein, und pflanzliche Produkte, zum Beispiel Mehle.

An oberflächenaktiven Stoffen sind zu nennen zum Beispiel Calciumligninsulfonat, Polyethylenalkylphenylether, Naphthalinsulfonsäuren und deren Salze, Phenolsulfonsäuren und deren Salze, Formaldehydkondensate, Fettalkoholsulfate sowie substituierte Benzolsulfonsäuren und deren Salze.

Der Anteil des beziehungsweise der Wirkstoffe(s) in den verschiedenen Zubereitungen kann in weiten Grenzen variieren. Beispielsweise enthalten die Mittel etwa 10 bis 90 Gewichtsprozent Wirkstoff, etwa 90 bis 10 Gewichtsprozent flüssige oder feste Trägerstoffe sowie gegebenenfalls bis zu 20 Gewichtsprozent oberflächenaktive Stoffe.

Die Ausbringung der Mittel kann in üblicher Weise erfolgen, zum Beispiel mit Wasser als Träger in Spritzbrühmengen etwa 100 bis 1000 Liter/ha. Eine Anwendung der Mittel im sogenannten Low-Volume und Ultra-Low-Volume-Verfahren ist ebenso möglich wie ihre Applikation in Form von sogenannten Mikrogranulaten.

Die Herstellung dieser Zubereitungen kann in an sich bekannter Art und Weise, zum Beispiel durch Mahl- oder Mischverfahren, durchgeführt werden. Gewünschtenfalls können Zubereitungen der Einzelkomponenten auch erst kurz vor ihrer Verwendung gemischt werden, wie es zum Beispiel im sogenannten Tankmixverfahren in der Praxis durchgeführt wird.

Zur Herstellung der verschiedenen Zubereitungen werden zum Beispiel die folgenden Bestandteile eingesetzt:

| A) **Spritzpulver** | | | |
|---|---|---|---|
| 1.) | 25 | Gewichtsprozent | Wirkstoff |
| | 60 | Gewichtsprozent | Kaolin |
| | 10 | Gewichtsprozent | Kieselsäure |
| | 5 | Gewichtsprozent | einer Mischung aus dem Calciumsalz der Ligninsulfonsäure und dem Natriumsalz des N-Methyl-N-oleyl-taurins |
| 2.) | 40 | Gewichtsprozent | Wirkstoff |
| | 25 | Gewichtsprozent | Tonmineralien |
| | 25 | Gewichtsprozent | Kieselsäure |
| | 10 | Gewichtsprozent | einer Mischung aus dem Calciumsalz der Ligninsulfonsäure und Alkylphenylpolyglycolethern |

| B) **Paste** | | |
|---|---|---|
| 45 | Gewichtsprozent | Wirkstoff |
| 5 | Gewichtsprozent | Natriumaluminiumsilikat |
| 15 | Gewichtsprozent | Cetylpolyglycolether mit 8 Mol Ethylenoxid |
| 2 | Gewichtsprozent | Spindelöl |
| 10 | Gewichtsprozent | Polyethylenglycol |
| 23 | Gewichtsprozent | Wasser |

| C) **Emulsionskonzentrat** | | |
|---|---|---|
| 25 | Gewichtsprozent | Wirkstoff |
| 15 | Gewichtsprozent | Cyclohexanon |
| 55 | Gewichtsprozent | Xylol |
| 5 | Gewichtsprozent | einer Mischung aus dem Calciumsalz der Dodecylbenzolsulfonsäure und Nonylphenylpolyoxyethylen |

| D) **Granulat** | | |
|---|---|---|
| 0,83 | Gewichtsprozent | Wirkstoff |
| 99,17 | Gewichtsprozent | Attapulgitgranulat |

Die folgenden Beispiele erläutern die Herstellung der erfindungsgemäßen Verbindungen:

**Beispiel 1**

N-(3-Fluorphenyl)-4-methylen-1,3-oxathiolan-2-imin

3,9 ml Propargylalkohol und eine Spatelspitze Natriummethanolat in 30 ml Diethylether werden bei 10°C mit einer Lösung aus 10 g 3-Fluorphenylisothiocyanat in 70 ml Diethylether versetzt. Nach einer Stunde Rühren bei Raumtemperatur und zwei Stunden unter Rückfluß wird auf Wasser gegeben und mit Diethylether extrahiert. Die organische Phase wird mit Wasser gewaschen, über Magnesiumsulfat getrocknet und eingeengt. Das Rohprodukt wird durch Säulenchromatographie an Kieselgel (Elutionsmittel: Methylenchlorid) gereinigt.
Ausbeute: 7,2 g = 53 % der Theorie
$n_D^{20}$ : 1,6039

Analog dem oben angegebenen Verfahren wurden die in der folgenden Tabelle aufgeführten Verbindungen hergestellt:

| Beispiel | X | Y | Z | $R^1$ | $R^2$ | $R^3$ | Fp | $n_D^{20}$ |
|---|---|---|---|---|---|---|---|---|
| 2 | 2-F | 4-Cl | 5-$OCH_3$ | H | H | H | | Harz |
| 3 | 2-F | 4-Cl | 5-$OCH_3$ | $CH_3$ | $CH_3$ | H | | Harz |
| 4 | 2-F | H | H | H | H | H | | 1,6062 |
| 5 | 2-Cl | H | H | H | H | H | | 1,6281 |
| 6 | 3-Cl | H | H | H | H | H | | 1,6322 |
| 7 | 4-Cl | H | H | H | H | H | 53 | |
| 8 | 4-F | H | H | H | H | H | 46 | |
| 9 | 2-Cl | 4-Cl | H | H | H | H | 81 | |
| 10 | 3-$CF_3$ | H | H | H | H | H | | 1,5488 |
| 11 | 3-$CF_3$ | H | H | H | H | $CH_3$ | | 1,5462 |
| 12 | 3-$CF_3$ | H | H | $CH_3$ | H | H | | 1,5384 |
| 13 | 3-$CF_3$ | H | H | $CH_3$ | $CH3_3$ | H | | 1,5290 |
| 14 | 3-$CF_3$ | 4-$NO_2$ | H | H | H | H | 63 | |
| 15 | 2-Cl | 5-$CF_3$ | H | H | H | H | | 1,5582 |
| 16 | 3-$CF_3$ | 4-Cl | H | H | H | H | | 1,5760 |
| 17 | 3-$CF_3$ | 4-F | H | H | H | H | | 1,5436 |
| 18 | 2-F | 5-$CF_3$ | H | H | H | H | | 1,5423 |
| 19 | 3-$CF_3$ | 5-$CF_3$ | H | H | H | H | | 1,5059 |

Die folgenden Beispiele erläutern die Anwendungsmöglichkeiten einer erfindungsgemäßen Verbindung:

**Beispiel A**

Im Gewächshaus wurden die aufgeführten Pflanzenspezies vor dem Auflaufen mit den aufgeführten Verbindungen in einer Aufwandmenge von 3,0 kg Wirkstoff/ha behandelt. Die Verbindungen wurden zu diesem Zweck als Emulsionen oder Suspensionen mit 500 Litern Wasser/ha gleichmäßig über die Pflanzen versprüht. Hier zeigen drei Wochen nach der Behandlung die erfindungsgemäßen Verbindungen eine hohe Kulturpflanzen-Selektivität in Baumwolle, Sonnenblume, Weizen und Mais bei ausgezeichneter Wirkung gegen das Unkraut. Das Vergleichsmittel zeigte nicht die gleichhhohe Selektivität.

In der folgenden Tabelle bedeuten:

0 = keine Schädigung
1 = 1 - 24 % Schädigung
2 = 25 - 74 % Schädigung
3 = 75 - 89 % Schädigung
4 = 90 - 100 % Schädigung
\- = nicht geprüft
GOSHI = Gossypium hirsutum
HELAN = Helianthus annuus
TRZAX = Triticum aestivum
ZEAMX = Zea mays
ALOMY = Aldpecurus myosuroides
SETVI = Setaria viridis
PANSS = Panicum maximum
SORHA = Sorghum halepense
MATCH = Matricaria chamomilla
VIOSS = Viola sp.

| Erfindungsgemäße Verbindungen | GOSHI | HELAN | TRZAX | ZEAMX | ALOMY | SETVI | PANSS | SORHA | MATCH | VIOSS |
|---|---|---|---|---|---|---|---|---|---|---|
| Beispiel 1 | 0 | 1 | 0 | 0 | 4 | 4 | 4 | 4 | 4 | - |
| Beispiel 6 | 0 | 0 | 0 | 0 | 4 | 4 | 4 | 4 | 4 | 3 |
| Beispiel 10 | 0 | 0 | 0 | 0 | 4 | 4 | 4 | 4 | 4 | 3 |
| Unbehandelt | 0 | 0 | 0 | 0 | 0 | 0 | 0 | 0 | 0 | 0 |
| <u>Vergleichsmittel</u> | | | | | | | | | | |
| Supertone | - | - | 3 | 2 | 4 | 4 | 4 | 4 | 4 | - |

**Beispiel B**

Im Gewächshaus wurden die aufgeführten Pflanzenspezies nach dem Auflaufen mit den aufgeführten Verbindungen in einer Aufwandmenge von 3,0 kg Wirkstoff/ha behandelt. Die Verbindungen wurden zu diesem Zweck als Emulsionen oder Suspensionen mit 500 Litern Wasser/ha gleichmäßig über die Pflanzen versprüht. Hier zeigen zwei Wochen nach der Behandlung die erfindungsgemäßen Verbindungen eine hohe Kulturpflanzen-Selektivität in Weizen und Gerste bei ausgezeichneter Wirkung gegen das Unkraut.

In der folgenden Tabelle bedeuten:

0 = keine Schädigung
1 = 1 - 24 % Schädigung
2 = 25 - 74 % Schädigung
3 = 75 - 89 % Schädigung
4 = 90 - 100 % Schädigung
HORVX = Hordeum vulgare
TRZAX = Triticum aestivum
SETVI = Setaria viridis
VERPE = Veronica persica
VIOSS = Viola sp.

| Erfindungsgemäße Verbindungen | HORVX | TRZAX | SETVI | VERPE | VIOSS |
|---|---|---|---|---|---|
| Beispiel 6 | 1 | 1 | 3 | 4 | 4 |
| Beispiel 10 | 1 | 1 | 3 | 4 | 3 |
| Unbehandelt | 0 | 0 | 0 | 0 | 0 |

**Beispiel C**

Im Gewächshaus wurde die in der Tabelle aufgeführte Verbindung mit der ebenfalls erwähnten Aufwandmenge appliziert. Hierzu wurde der Wirkstoff in ein Gefäß mit 1500 ml Wasser gegeben. Als Testpflanzenarten wurden Oryzasativa (ORYSA) und Scirpus juncoides (SCPJU) im 2- bis 5-Blatt-Stadium eingesetzt. Drei Wochen nach der Applikation wurde die Schädigung der Pflanzen bonitiert, und zwar nach folgendem Schema:
0 = keine Schädigung
1 = schwache Schädigung
2 = mittlere Schädigung
3 = starke Schädigung
4 = total vernichtet

| Erfindungsgemäße Verbindung | Wasserapplikation ppm | ORYSA | SCPJU |
|---|---|---|---|
| Beispiel 10 | 1 | 0 | 4 |

Wie die Tabelle zeigt, ist die erfindungsgemäße Verbindung stark wirksam gegen wichtige Reisunkräuter und gleichzeitig selektiv zu Wasserreis.

**Beispiel D**

Im Gewächshaus wurde die in der Tabelle aufgeführte erfindungsgemäße Verbindung mit der ebenfalls erwähnten Aufwandmenge appliziert. Hierzu wurde der Wirkstoff als Granulat auf die Bodenoberfläche gestreut. Als Testpflanzen wurden Oryza sativa (ORYSA), Eleocharis acicularis (ELOAC) und Monochoria vaginalis (MOOVA) im 2- bis 5-Blatt-Stadium eingesetzt. Drei Wochen nach der Applikation wurde die Schädigung der Pflanzen bonitiert, und zwar nach folgendem Schema:
0 = keine Schädigung
1 = schwache Schädigung
2 = mittlere Schädigung

3 = starke Schädigung
4 = total vernichtet

| Erfindungsgemäße Verbindung | kg Wirkstoff/ha | ORYSA | ELOAC | MOOVA |
|---|---|---|---|---|
| Beispiel 10 | 1,0 | 1 | 4 | 4 |
| Beispiel 16 | 1,0 | 0 | 4 | 4 |
| **Vergleichsmittel** | | | | |
| Pyrazolate | 1,0 | 1 | 2 | 1 |

Wie die Tabelle zeigt, sind die erfindungsgemäßen Verbindungen stark wirksam gegen wichtige Reisunkräuter und gleichzeitig selektiv zu Wasserreis. Das Vergleichsmittel hat nicht die gleichstarke Unkrautwirkung.

## Ansprüche

1. Substituierte N-Phenyl-4-alkyliden-1,3-oxathiolan-2-imine der allgemeinen Formel I

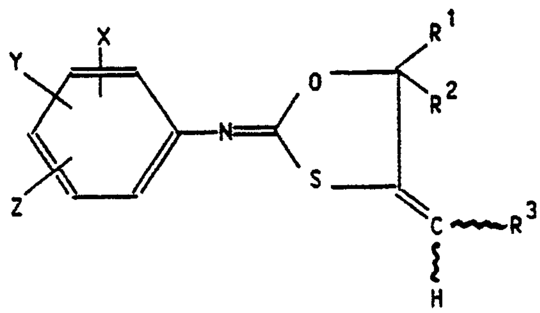

(I) ,

in der
X ein Halogenatom oder einen Halogen-$C_1$-$C_4$-alkylrest,
Y ein Wasserstoffatom, einen $C_1$-$C_4$-Alkylrest, einen Halogen-$C_1$-$C_4$-alkylrest, einen $C_1$-$C_4$-Alkoxyrest, einen Halogen-$C_1$-$C_4$-alkoxyrest, ein Halogenatom oder eine Nitrogruppe,
Z ein Wasserstoffatom, einen $C_1$-$C_4$-Alkylrest, einen Halogen-$C_1$-$C_4$-alkylrest, einen $C_1$-$C_4$-Alkoxyrest, einen Halogen-$C_1$-$C_4$-alkoxyrest, ein Halogenatom oder eine Nitrogruppe,
$R^1$ ein Wasserstoffatom, einen $C_1$-$C_4$-Alkylrest, einen Halogen-$C_1$-$C_4$-alkylrest oder einen Phenylrest,
$R^2$ ein Wasserstoffatom, einen $C_1$-$C_4$-Alkylrest, einen Halogen-$C_1$-$C_4$-alkylrest oder einen Phenylrest oder
$R^1$ und $R^2$ gemeinsam mit dem benachbarten Kohlenstoffatom einen carbocyclischen $C_3$-$C_6$-Ring bilden und
$R^3$ ein Wasserstoffatom, einen $C_1$-$C_4$-Alkylrest, einen Halogen-$C_1$-$C_4$-alkylrest oder einen Phenylrest bedeuten,
und deren Salze mit anorganischen und organischen Säuren.

2. Verfahren zur Herstellung von substituierten N-Phenyl-4-alkyliden-1,3-oxathiolan-2-iminen der allgemeinen Formel I, dadurch gekennzeichnet, daß man Verbindungen der allgemeinen Formel II

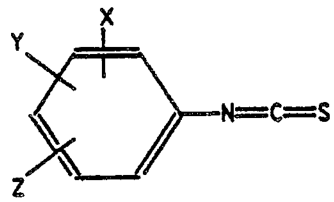

(II) ,

in der X, Y und Z die unter der allgemeinen Formel I genannten Bedeutungen haben, mit Verbindungen der allgemeinen Formel III

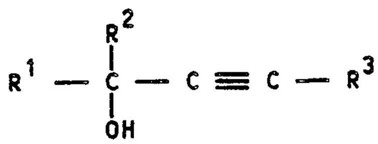

(III) ,

in der $R^1$, $R^2$ und $R^3$ die unter der allgemeinen Formel I genannten Bedeutungen haben, unter Verwendung eines basischen Katalysators umsetzt.

3. Mittel mit herbizider Wirkung, gekennzeichnet durch einen Gehalt an mindestens einer Verbindung gemäß dem Anspruch 1.

4. Verwendung von Mitteln gemäß dem Anspruch 3 zur Bekämpfung monokotyler und dikotyler Unkrautarten in landwirtschaftlichen Hauptkulturen.

5. Verfahren zur Herstellung von Mitteln mit herbizider Wirkung, dadurch gekennzeichnet, daß man Verbindungen der allgemeinen Formel I gemäß dem Anspruch 1 mit Träger- und/oder Hilfsstoffen vermischt.